# EUROPÄISCHE PATENTSCHRIFT

(11) **EP 3 975 941 B1**
(45) Veröffentlichungstag und Bekanntmachung des Hinweises auf die Patenterteilung: **20.03.2024**
(21) Anmeldenummer: 20727971.2
(22) Anmeldetag: 20.05.2020
(51) Int. Cl.: A61F 2/78, A61F 2/50, A61F 2/80

(54) **VERFAHREN ZUM HERSTELLEN EINES PROTHESENLINERS UND SYSTEM AUS PROTHESENLINER UND PROTHESENSCHAFT**
METHOD FOR PRODUCING A PROSTHETIC LINER AND SYSTEM CONSISTING OF PROSTHETIC LINER AND PROSTHETIC SOCKET
PROCÉDÉ DE FABRICATION D'UN MANCHON DE PROTHÈSE ET SYSTÈME COMPRENANT UN MANCHON DE PROTHÈSE ET UNE EMBOÎTURE DE PROTHÈSE

(30) Priorität: 29.05.2019 DE 102019114461
(43) Veröffentlichungstag der Anmeldung: 06.04.2022
(73) Patentinhaber: Ottobock SE & Co. KGaA, 37115 Duderstadt (DE)
(72) Erfinder: FINKE, Lars Benjamin, 37136 Landolfshausen (DE)
(74) Vertreter: Gramm, Lins & Partner Patent- und Rechtsanwälte PartGmbB
(86) Internationale Anmeldenummer: PCT/EP2020/064059
(87) Internationale Veröffentlichungsnummer: WO 2020/239571

(56) Entgegenhaltungen:
- EP-A1- 3 238 667
- EP-A1- 3 298 991
- DE-A1- 10 142 491
- DE-A1-102013 010 371
- DE-A1-102016 114 681
- DE-A1-102017 108 913

## Beschreibung

Die Erfindung betrifft ein Verfahren zum Herstellen eines Prothesenliners zum Einsatz in einem Prothesenschaft, der einen Aufnahmeraum mit einem distalen Ende und einem proximalen Rand für einen Stumpf eines Patienten und den Prothesenliner aufweist ein System aus einem Prothesenschaft und einem Prothesenliner sowie einen Prothesenliner als solchen.

Prothesen ersetzen nicht vorhandene oder nicht mehr vorhandene Gliedmaße hinsichtlich der Funktion und/oder deren Erscheinung. Die Prothese selbst wird an einem Stumpf angeordnet und daran festgelegt. Zur Festlegung existieren unterschiedliche Systeme, ein Befestigungssystem sieht die sogenannte Vakuumschafttechnologie vor, bei der im angelegten Zustand das Volumen zwischen dem Stumpf und einer Prothesenschaftinnenwand evakuiert wird. Zur Abdichtung und zur Abpolsterung kann an dem Stumpf ein sogenannter Prothesenliner angeordnet sein, der in der Regel ein geschlossenes distales Ende und eine proximale Einstiegsöffnung aufweist und den Stumpf umgibt. Zwischen der Lineraußenwand und der Prothesenschaftinnenwand wird durch das Einführen des mit dem Prothesenliner versorgten Stumpfes ein Volumen gebildet, das evakuiert wird, wodurch eine kraftschlüssige Verbindung zwischen dem Prothesenschaft und dem Prothesenliner entsteht. Der Prothesenliner haftet an dem Prothesenstumpf über Haftkräfte, sodass der Prothesenschaft und die an dem Prothesenschaft befestigten Komponenten an dem Stumpf des Patienten festgelegt sind. Um eine dauerhafte Befestigung des Prothesenschaftes zu erreichen, ist es notwendig, das Volumen gegenüber der Atmosphäre abzudichten. Dazu sind sogenannte Kappen oder Manschetten vorgesehen, die über den proximalen Rand des Prothesenschaftes gezogen werden und an der Außenseite des Prothesenliners oder des Stumpfes anliegen, sodass in den Spalt zwischen dem proximalen Rand des Prothesenschaftes und dem Prothesenliner oder dem Stumpf keine Luft eintreten kann. Alternativ zu einer Manschette oder auch Kappe können Dichtlippen an der Außenseite des Liners oder an der Innenseite des Prothesenschaftes angeordnet oder festgelegt sein, um eine Abdichtung eines Volumens zu bewirken.

Der Prothesenschaft besteht in der Regel aus einem formstabilen Material, um eine ausreichende Stabilität und Festigkeit zu haben, um weitere Prothesenkomponenten daran anzuordnen und eine Stützfunktion für die Weichteilkomponenten bereitzustellen. Der proximale Rand des Prothesenschaftes ist dabei möglichst hochgezogen ausgebildet, um den Gliedmaßenstumpf sicher aufnehmen zu können. Bei einem Unterschenkelschaft ragt der proximale Rand medial und lateral bis zu den Kniekondylen und ist im Schienbeinbereich und im Kniekehlenbereich tief ausgeschnitten. Eine ähnliche Konstruktion ergibt sich bei einem Unterarmschaft. Bei einem Oberschenkelschaft ist eine laterale Erhöhung ausgebildet, um eine seitliche Stabilität bereitzustellen. Prothesenschäfte sind in der Regel individuell angefertigte Produkte, die korrespondierend zu der Stumpfform hergestellt werden. Prothesenliner sind häufig Standardprodukte aus elastischem Material, die sich an unterschiedliche Stumpfformen anpassen können. Um möglichst unterschiedliche Stumpflängen mit vorkonfektionierten Prothesenlinern versorgen zu können, werden Dichtlippen am Liner üblicherweise relativ weit distal oder verschieblich angeordnet, so das der proximale Anteil des Liners individuell gekürzt werden kann, ohne die Funktion des Dichtelementes zu beeinträchtigen. Die jeweilige Abdichtung zwischen Prothesenliner und Prothesenschaft ist daher in diesem Fall ein Kompromiss zwischen industrieller Herstellbarkeit und einem individuell möglichst großen, abzudichtenden Volumen im Schaft.

Aus der US 10 376 392 B2 ein System aus einem Prothesenschaft und einer Abdichthülse innerhalb des Prothesenschaftes sowie einer ringförmigen inneren Krempe, wobei die Krempe in einer Ausnehmung in dem Prothesenschaft angeordnet ist. Die Ausnehmung oder Nut kann der Kontur des oberen Randes des Prothesenschaftes folgend ausgebildet sein.

Die WO 2018/015736 A1 betrifft eine Dichtung für einen Prothesenliner mit einem schlauchartigen Träger und einer oder mehrerer Dichtlippen, die radial von dem Träger abstehen und um ihn herum verlaufen. Zumindest eine der Dichtlippen verläuft in Axialrichtung wellenförmig um den Träger. Sie betrifft ebenfalls einen Prothesenliner, der zumindest eine in Axialrichtung wellenförmig verlaufende, umlaufende Dichtlippe aufweist. Das Dokument EP 3 298 991 A1 zeigt ein System aus einem Prothesenschaft und einem Prothesenliner gemäß dem Oberbegriff des Anspruchs 12.

Aufgabe der vorliegenden Erfindung ist es, ein Verfahren zum Herstellen eines Prothesenliners und ein System aus Prothesenschaft und Prothesenliner bereitzustellen, mit denen es möglich ist, eine sichere Festlegung des Prothesenschaftes an einen Stumpf zu ermöglichen.

Erfindungsgemäß wird diese Aufgabe durch ein Verfahren mit den Merkmalen des Hauptanspruches und ein System mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zum Herstellen eines Prothesenliners zum Einsatz in einem Prothesenschaft, der einen Aufnahmeraum mit einem distalen Ende und einem proximalen Rand für einen Stumpf und den an dem Stumpf angelegten Prothesenliner aufweist, sieht vor, dass ein Dichtlippenverlauf an der Außenseite des Prothesenliners korrespondierend zu dem Verlauf einer Höhenkontur des proximalen Randes des Prothesenschaftes oder anhand vorhandener, bekannter anatomischer Gegebenheiten des Stumpfes festgelegt wird, wobei die Dichtlippe einen Dichtlippenverlauf in Gestalt einer Raumkurve ausbildet. Entlang des festgelegten Dichtlippenverlaufes wird eine Dichtlippe an der Außenseite des Prothesenliners angeordnet. Der Dichtlippenverlauf folgt somit im angelegten Zustand der Höhenkontur des proximalen Randes des Prothesenschaftes und ist korrespondierend zu der oberen Abschlussgeometrie des Prothesenschaftes ausgebildet. Die Position der Dichtlippe an dem Prothesenliner ist dabei so festgelegt, dass die Dichtlippe distal zu dem proximalen Rand des Prothesenschaftes angeordnet ist, wenn der an den Prothesenstumpf angelegte Prothesenliner vollständig in den Prothesenschaft eingeführt ist. Durch den korrespondierenden Verlauf der Dichtlippe zu der proximalen Abschlussgeometrie des Prothesenschaftes ist es möglich, die Dichtlippe im angezogenen Zustand des Prothesenliners maximal weit proximal in dem Prothesenschaft anzuordnen und somit eine maximale abgedichtete Oberfläche des Volumens zu erreichen. Über die maximierte Fläche distal zu der Dichtlippe ist es möglich, eine maximale Kraft bei möglichst großflächiger und gleichmäßiger Kraftverteilung zum Halten des Prothesenschaftes an dem Prothesenliner aufzubringen. Durch die individuell an den proximalen Rand des Prothesenschaftes angepasste Geometrie und einen entsprechenden Verlauf der Dichtlippe wird die mechanische Güte des Interfaces maximiert und die Belastungen auf den Stumpf, insbesondere den Amputationsstumpf, reduziert. Dies erhöht den Komfort für den Anwender und stellt eine sichere Verbindung zwischen dem Prothesenschaft und dem Stumpf her. Der Dichtlippenverlauf kann z.B. anhand eines vorhandenen oder errechneten, insbesondere in digitaler Form vorliegenden Verlaufes des proximalen Randes eines Prothesenschaftes festgelegt werden. Der bekannte Verlauf des proximalen Randes des Prothesenschaftes dient als Referenz für den Dichtlippenverlauf, der entsprechend festgelegt wird. Ebenso kann die Festlegung des Dichtlippenverlaufes aufgrund eines gescannten Stumpfes oder anderweitig erhaltener Daten über die Form und/oder Beschaffenheit des Stumpfes oder dessen anatomischer Gegebenheiten erfolgen. Die digital vorliegende oder errechnete Anatomie kann als Grundlage für den zu fertigenden Prothesenliner und zudem für den zu fertigenden Prothesenschaft dienen. Der Prothesenliner und ggf. auch der Prothesenschaft wird digital um das Modell oder digitale Abbild des Stumpfes herum modelliert. Die Form korrespondiert im Wesentlichen zu der Außenkontur des Stumpfes, mit Zuschlägen für Polster an dem Liner und ggf. Anpassungen für den Prothesenschaft für Entlastungsbereiche oder Kompressionsbereiche zum Ausgleichen von Volumenschwankungen. Der Prothesenliner kann ebenfalls nur anhand des Stumpfmodells oder des digitalen Abbildes des Stumpfes und/oder des Prothesenschaftes entworfen und in einen digitalen Datensatz umgewandelt werden, wobei der Dichtlippenverlauf anhand der anatomischen Gegebenheiten ohne bereits vorhandenen oder errechneten Prothesenschaftdatensatz bestimmt werden kann.

In einer Weiterbildung der Erfindung wird vor dem Festlegen des Dichtlippenverlaufes die Höhenkontur des proximalen Randes des Prothesenschaftes erfasst. Die Höhenkontur, also der Verlauf in Proximal-Distal-Richtung des proximalen Randes des Prothesenschaftes, bildet den Verlauf der oberen Abschlussgeometrie des Prothesenschaftes. Neben der Erfassung einer Höhenkontur eines bereits physisch vorhandenen Prothesenschaftes, beispielsweise durch ein Abtastverfahren, Entlangfahren eines Höhensensors auf dem proximalen Rand oder parallel dazu und Zuordnen der Höhendaten zu den Umfangskoordinaten oder durch eine berührungslose Messung, beispielsweise optische Messverfahren oder anderweitiges Scannen, kann die Festlegung auch auf der Grundlage allein von Daten über einen Prothesenschaft, insbesondere eines noch zu fertigenden Prothesenschaftes, erfolgen. Existiert bereits ein 3D-Modell oder ein Datensatz, anhand dessen der Prothesenschaft gefertigt werden soll, kann der Dichtlippenverlauf auf der Grundlage dieser Daten festgelegt und eine Fertigung des Prothesenliners erfolgen.

In einer Weiterbildung der Erfindung ist es vorgesehen, dass der Prothesenliner additiv gefertigt wird, beispielsweise im Rahmen eines Rapid-Liquid-Printing-Verfahrens, um eine schnelle und einstückige sowie individuelle Anpassung des Prothesenliners an die jeweilige Höhenkontur und gegebenenfalls Umfangskontur des Prothesenschaftes vornehmen zu können. Das Rapid-Liquid-Printing-Verfahren ist beispielsweise in der US 2018-281295 A1 beschrieben. Alternativ kann die Dichtlippe entlang des festgelegten Dichtlippenverlaufes an der Außenseite des Prothesenliners dauerhaft festgelegt werden, beispielsweise verklebt, verschweißt, angegossen oder vernetzt oder in einem additiven Fertigungsverfahren an der Außenseite des Liners festgelegt werden. Im Bereich der Kontaktfläche zwischen der Dichtlippe und einem Grundkörper des Prothesenliners findet eine stoffschlüssige, insbesondere luftundurchlässige Verbindung statt. Die Dichtlippe kann separat ausgebildet und anschließend entlang des festgelegten Dichtlippenverlaufes aufgebracht und festgelegt werden, alternativ kann ein Angießen und somit Anformen oder die additive Fertigung der Dichtlippe vorzugsweise an einen noch nicht vollständig vernetzten Grundkörper entlang des Dichtlippenverlaufes erfolgen. Der Dichtlippenverlauf kann an dem Prothesenliner angezeichnet oder durch eine Erhebung oder eine Vertiefung entlang des Dichtlippenverlaufes angezeigt werden, um eine Positionierhilfe für eine separate, zu fixierende oder anzuformende oder additiv festzulegende Dichtlippe bereitzustellen.

Die Dichtlippe kann in distaler Richtung zu dem proximalen Rand des Prothesenschaftes versetzt an dem Prothesenliner angeordnet sein, um zu vermeiden, dass im angezogenen, vollständig eingeführten Zustand des Prothesenliners die Dichtlippe proximal über den proximalen Rand des Prothesenschaftes hinaussteht. Durch die Verschiebung oder Verlagerung in distaler Richtung von dem proximalen Rand des Prothesenschaftes weg wird eine Sicherheitszone eingerichtet, durch die beispielsweise Abweichungen in der vorgesehenen Orientierung des Prothesenliners an dem Stumpf ausgeglichen werden können.

Der Verlauf der Höhenkontur erfolgt in Proximal-Distal-Richtung und berücksichtigt die Gesamtlänge des Prothesenschaftes und damit auch die Entfernung des Dichtlippenverlaufes von dem distalen Ende des Prothesenliners. Alternativ oder ergänzend kann auch die Umfangskontur über den Umfang erfasst werden, um unterschiedliche Höhen der Dichtlippe, also der radiale Abstand von der Dichtlippenaußenkante bis zu dem Grundkörper des Prothesenliners, an den jeweiligen Patienten anpassen zu können.

Die Dichtlippe kann über den Umfang des Prothesenliners in Proximal-Distal-Richtung gleichbeabstandet zu dem proximalen Rand des Prothesenschaftes angeordnet werden, also im Wesentlichen identisch zu der Höhenkontur des proximalen Randes des Prothesenschaftes verlaufen.

Die Dichtlippe kann in einem Dichtlippenbereich angeordnet werden, der breiter als die Dichtlippe selbst ist und einen Montagebereich darstellt, innerhalb dessen eine separate Dichtlippe angeordnet und festgelegt werden kann. Der Dichtlippenbereich dient zur Erleichterung der Fertigung und ermöglicht es auch, in einem additiven Fertigungsverfahren die Dichtlippe selbst innerhalb eines vorgegebenen Bereiches in Proximal-Distal-Richtung anzuordnen, anzugießen, anzuformen oder auszubilden. Die proximale und distale Grenze des Dichtlippenbereiches korrespondiert zu der Höhenkontur des proximalen Randes des Prothesenschaftes.

Der Dichtlippenbereich kann beispielsweise doppelt so breit wie die Dichtlippe an ihrem Übergang zu einem Prothesenlinergrundkörper sein.

Die Höhenkontur oder die Höhenkontur und die Umfangskontur des Prothesenschaftes kann optisch erfasst werden. Die erfassten Bilddaten bilden eine Basis für ein digitales 3D-Modell, für das oder von dem ein Datensatz erstellt wird. Aufgrund des Datensatzes des 3D-Modells werden der Dichtlippenverlauf oder der Dichtlippenverlauf und die Dichtlippenform in Abhängigkeit von der erfassten Höhenkontur oder der erfassten Umfangs- und Höhenkontur festgelegt. Die Erfassung der Höhenkontur kann auch aus bereits vorliegenden Daten, etwa einem 3D-Modell des Schaftes, erfolgen, ohne dass ein Prothesenschaft physisch vorhanden sein muss. Wird beispielsweise ein Prothesenschaft in einem additiven oder in einem anderen Fertigungsverfahren auf der Grundlage eines Datensatzes eines Gliedmaßenstumpfes, beispielsweise eines Amputationsstumpfes, erstellt, folgt die Innenkontur des Prothesenschaftes im Wesentlichen der Außenkontur des Stumpfes mit einer Zugabe für Volumenschwankungen und gegebenenfalls die Materialstärke des Prothesenliners. Wird der Prothesenschaft nicht auf der Basis von digitalen Daten, die beispielsweise von dem Stumpf selbst oder einem Gipsmodell davon abgenommen werden, hergestellt, kann die Innenkontur eines bereits vorhandenen Prothesenschaftes optisch oder auf andere Art und Weise erfasst und in einem Computersystem gespeichert werden. Anhand des Vergleiches der Außenkontur des Stumpfes und der Innenkontur des Prothesenschaftes und der ebenfalls im Rahmen des 3D-Modells vorhandenen Höhenkontur des Prothesenschaftrandes am proximalen Ende werden dann die Position und der Verlauf der Dichtlippe an der Außenseite des Prothesenliners festgelegt. Ebenfalls kann die Höhe und die Form sowie die Dicke des Prothesenliners bzw. Prothesenlinergrundkörpers festgelegt und als Datensatz zur Fertigung in einem additiven Fertigungsverfahren verarbeitet werden. Im Rahmen des additiven Fertigungsverfahrens, beispielsweise im Rahmen eines Rapid-Liquid-Printing-Verfahrens kann dann der Prothesenliner mit dem an die Höhenkontur und/oder Umfangskontur angepassten Dichtlippenverlauf und Dichtlippenhöhe und/oder Dichtlippendicke hergestellt werden. Der Verlauf der Dichtlippe kann auch direkt aus anatomischen Daten bestimmt werden, etwa auf Grundlage eines Scans eines Stumpfes. Die Höhenkontur und die Umfangskontur des Schaftes können dann entweder aus dem zunächst als geeignet oder optimal erachteten Verlauf der Dichtlippe bestimmt werden, so dass der Dichtlippenverlauf als Bezugsgröße für den Verlauf des proximalen Randes des Prothesenschaftes dient. Oder die Höhenkontur und die Umfangskontur des Schaftes werden ebenfalls aus den anatomischen Daten des Scans ermittelt. Im Ergebnis korrespondiert der Verlauf der Dichtlippe zu dem Verlauf der Höhenkontur des Prothesenschaftes, unabhängig davon ob der Dichtlippenverlauf in Abhängigkeit der zuerst festgelegten Höhenkontur des Prothesenschaftes, die Höhenkontur des Prothesenschaftes in Abhängigkeit von dem zunächst bestimmten Dichtlippenverlauf oder der Dichtlippenverlauf und die Höhenkontur unabhängig voneinander auf der Grundlage der anatomischen Gegebenheiten anhand z.B. des digitalen 3D-Stumpfmodells erstellt und festgelegt werden.

Die Dichtlippenhöhe kann in Abhängigkeit von einem erfassten Abstand zwischen einem Innenumfang des Prothesenschaftes und einem Außenumfang des einzuführenden Stumpfes festgelegt werden.

Das System aus einem Prothesenschaft und einem Prothesenliner sieht vor, dass der Prothesenschaft einen Aufnahmeraum für einen mit dem Prothesenliner versorgten Stumpf aufweist. Der Prothesenschaft weist ein distales Ende und einen proximalen Rand auf. Der proximale Rand des Prothesenschaftes weist eine Höhenkontur und eine Umfangskontur auf. An der dem Prothesenschaft zugewandten Außenseite des Prothesenliners ist zumindest eine Dichtlippe ausgebildet oder festgelegt, wobei der Dichtlippenverlauf der zumindest einen Dichtlippe in dem vollständig eingeführten Zustand des Prothesenliners mit dem Verlauf der Höhenkontur des Prothesenschaftes korrespondiert, wobei die Dichtlippe einen Dichtlippenverlauf in Gestalt einer Raumkurve ausbildet. Die Dichtlippe muss nicht bündig mit dem proximalen Rand des Prothesenschaftes abschließen, vielmehr ist vorgesehen, dass die Dichtlippe der Abschlusskontur folgend distal vom proximalen Rand des Prothesenschaftes versetzt an dem Prothesenliner angeordnet ist.

Der Prothesenschaft ist distal zu der zumindest einen Dichtlippe im vollständig eingeführten Zustand des angelegten Prothesenliners geschlossenwandig ausgebildet, um einen möglichst großflächigen Interfacebereich herzustellen, sodass auf einer möglichst großen Fläche ein entsprechender Unterdruck erzeugt werden kann.

Der Prothesenschaft ist bevorzugt formstabil ausgebildet, um eine ausreichende Stabilität für die Aufnahme des Stumpfes mit dem Liner und die Anordnung weiterer Prothesenkomponenten, beispielsweise Prothesengelenke, bereitzustellen. Die zumindest eine Dichtlippe ist an einem Grundkörper des Prothesenliners befestigt oder ausgebildet und kann über den Umfang des Prothesenliners eine ungleichmäßige Höhe aufweisen, also radial nach außen unterschiedlich weit von einem Grundkörper abstehen, um Formschwankungen oder Formdifferenzen zwischen der Außenkontur des Stumpfes und der Innenkontur des Prothesenschaftes auszugleichen.

Eine Weiterbildung der Erfindung sieht vor, dass die zumindest eine Dichtlippe an einem Grundkörper des Prothesenliners befestigt oder ausgebildet ist und eine über den Umfang des Prothesenliners ungleichmäßige Breite aufweist, um Unterschiede in dem radialen Abstand zwischen der Außenseite des Grundkörpers im angelegten Zustand und der Innenseite des Prothesenschaftes ausgleichen zu können. Dadurch wird sichergestellt, dass die Dichtlippe immer an der Innenwand des Prothesenschaftes anliegt, wenn der Stumpf mit dem Liner eingeführt wird. Die über den Umfang des Prothesenliners unterschiedliche Dicke oder die unterschiedliche radiale Ausdehnung wird beispielsweise bei einem Vergleich zwischen der gescannten Innenseite des Prothesenschaftes und der gescannten Außenseite oder dem 3D-Modell des Stumpfes ermittelt.

Der Dichtlippenverlauf liegt bevorzugt nicht in einer Ebene und ist somit nicht geradlinig, sondern beschreibt eine Raumkurve mit einer unregelmäßigen Höhenkontur, also einem ungleichmäßigen Abstand zu dem distalen Ende des Prothesenliners oder des Prothesenschaftes über den Umfang.

Der Prothesenliner für ein oben beschriebenes System sieht zumindest eine an einer Außenseite des Prothesenliners ausgebildete oder festgelegte Dichtlippe vor, die einen Dichtlippenverlauf in Gestalt einer Raumkurve ausbildet. Der Dichtlippenverlauf der zumindest einen Dichtlippe ist zu dem Verlauf einer Höhenkontur eines proximalen Randes eines Prothesenschaftes, in den der an einen Stumpf angelegte Prothesenliner eingeführt werden soll, korrespondierend ausgebildet ist.

Nachfolgend wird ein Ausführungsbeispiel der Erfindung anhand der beigefügten Figuren näher erläutert. Es zeigen:
- Figur 1 -: einen Prothesenliner in Einzeldarstellung;
- Figur 2 -: ein System aus Prothesenschaft und einem daran angeordneten Prothesenliner; sowie
- Figur 3 -: eine Schemadarstellung einer Variante des Herstellverfahrens.

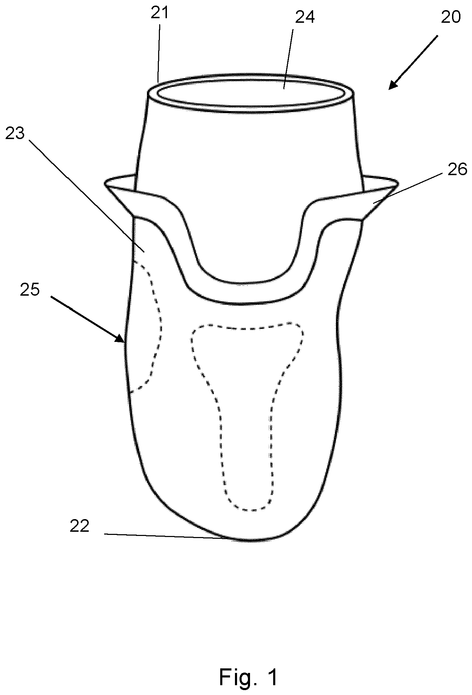

Figur 1 zeigt in einer Einzeldarstellung einen Prothesenliner 20 mit einem proximalen Rand 21 und einem distalen Endbereich 22. Der distale Endbereich 22 ist geschlossen ausgebildet, der proximale Rand 21 umgibt eine Einstiegsöffnung umfänglich. Der Prothesenliner 20 weist einen Grundkörper 25 mit einer Außenseite 23 und einer Innenseite 24 auf. Der Grundkörper 25 ist flexibel und bevorzugt elastisch, zumindest in Umfangsrichtung ausgebildet. Die Innenseite 24 des Grundkörpers 25 kann aus einem haftenden Polymer, beispielsweise Silikon bestehen. Alternativ dazu kann die Innenseite 24 mit einer haftenden Beschichtung vollständig und teilweise beschichtet sein. Die Beschichtung kann beispielsweise aus einem Silikon oder einem anderem, an der Haut haftenden Polymer ausgebildet sein. Die Außenseite 23 des Grundkörpers 25 kann ebenfalls aus einem Elastomer bestehen oder zumindest teilweise mit einem Elastomer beschichtet sein. Ebenfalls ist es möglich, dass an der Außenseite 23 ein Textil aufgebracht ist, um eine Vergleichmäßigung des Druckes in einem Zwischenraum zwischen dem Prothesenliner 20 und einem nicht dargestellten Prothesenschaft herzustellen. Alternativ oder ergänzend können an der Außenseite 23 Erhebungen oder Kanäle angeordnet sein, beispielsweise ausgebildet oder eingebracht bzw. aufgebracht, um strömungstechnische Verbindungen über die gesamte Längserstreckung, also von distal nach proximal, sowie um den Umfang herum zu ermöglichen.

An dem Grundkörper 25 ist eine Dichtlippe 26 angeordnet, die eine Abdichtung zwischen dem proximalen und dem distalen Bereich des Prothesenliners 20 im eingeführten Zustand in einen nicht dargestellten Prothesenschaft ausbildet. Die Dichtlippe 26 kann aus einem luftundurchlässigen Material hergestellt oder entsprechend beschichtet sein, dass kein Luftdurchtritt durch die Dichtlippe 26 erfolgt. Die Dichtlippe 26 kann beispielsweise aus einem Silikon oder einem Polymer bestehen oder mit einem solchen Werkstoff beschichtet sein. Eine Möglichkeit der Ausgestaltung der Dichtlippe 26 besteht darin, dass diese einstückig mit dem Grundkörper 25 ausgebildet wird, beispielsweise im Rahmen eines additiven Herstellverfahrens, beispielsweise über das Rapid Liquid Printing-Verfahren oder durch Angießen oder während eines anderen Urformverfahrens. Der Bereich distal der Dichtlippe 26 an der Außenseite 23 des Grundkörpers 25 kann mit einer strukturierten Oberfläche versehen sein, die eine Druckverteilung in voneinander beabstandeten Bereichen ermöglichen.

Die Strukturierung kann beispielsweise als Textilmaterial, das aufgeklebt oder aufkaschiert werden kann, oder über Kanäle und/oder Erhebungen an der Außenseite 23 erfolgen.

Die Dichtlippe 26 steht radial von dem Grundkörper 25 ab und ist bevorzugt elastisch ausgebildet, sodass die Dichtlippe 26 mit ihrer Außenseite, die von dem Grundkörper 25 weg weist, an dem Prothesenschaft anliegt und dagegen drückt. In dem dargestellten Ausführungsbeispiel ist die Dichtlippe 26 nicht senkrecht von der Außenseite 23 des Grundkörpers 25 abstehend ausgebildet, sondern geneigt ausgebildet oder angeordnet. Die Innenseite der Dichtlippe 26, die dem Grundkörper 25 zugewandt ist, schließt einen spitzen Winkel zwischen sich ein. Grundsätzlich ist es auch möglich, eine umgekehrt gerichtete Orientierung vorzusehen oder die Dichtlippe 26 senkrecht abstehen zu lassen. Beim Einführen des Prothesenliners 20 in einen Prothesenschaft wird dann die Dichtlippe 26 in der Regel umgeschlagen, sodass sich eine Orientierung ergibt, bei der die in distale Richtung orientierte Seite der Dichtlippe 26 an der Innenseite des Prothesenschaftes anliegt. Bei einem Unterdruck in dem durch die Dichtlippe 26 abgedichteten Volumen zwischen dem Prothesenschaft und dem distal zu der Dichtlippe 26 befindlichen Bereich gegenüber dem Atmosphärendruck, wird die Dichtlippe 26 gegen die Innenwand des Prothesenschaftes gedrückt, so dass sich ein selbstverstärkender Dichteffekt einstellt.

Der Figur 1 ist zu entnehmen, dass der proximale Rand 21 des Prothesenliners 20 geradlinig oder in einer Ebene angeordnet ausgebildet ist, wobei die Ebene im Wesentlichen senkrecht zu der Längserstreckung des Prothesenliners 20 verläuft. Davon abweichend verläuft die Dichtlippe 26 nicht in einer gemeinsamen Ebene, insbesondere nicht in einer Ebene parallel oder geneigt zu dem proximalen Rand 21 des Prothesenliners 20, sondern entlang einer Raumkurve, die korrespondierend zu dem Verlauf der Höhenkontur des Prothesenschaftes an seinem proximalen Rand verläuft. In dem dargestellten Ausführungsbeispiel der Figur 1 ist ein Prothesenliner 20 für einen Unterschenkel gezeigt. Die Dichtlippe 26 verläuft im frontalen Bereich knapp unterhalb der Patella und erstreckt sich medial und lateral in Richtung auf den proximalen Rand 21. Im rückwärtigen Teil des Prothesenliners kann die Dichtlippe 26 wieder in Distalrichtung abgesenkt verlaufen. Ein solcher Verlauf entspricht dem Verlauf des proximalen Randes eines Unterschenkelschaftes, der im frontalen Schienbeinbereich und im Kniekehlenbereich tiefer, also eher distal orientiert verläuft als medial-lateral. Medial und lateral des Kniegelenkes können Prothesenschaftbereiche weiter in Proximalrichtung verlaufend angeordnet sein, um eine erhöhte seitliche Stabilität und eine verbesserte Anlage des Unterschenkelschaftes an dem Stumpf zu erreichen.

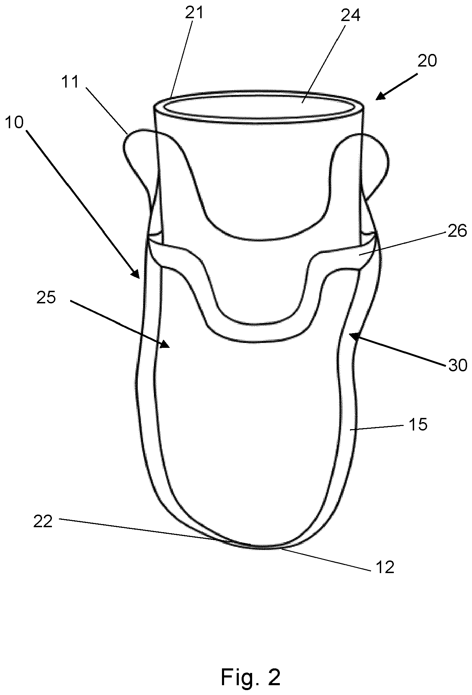

In Figur 2 ist in einer schematischen Darstellung der Prothesenliner 20 gemäß Figur 1 in einem angezogenen Zustand gezeigt. Der Prothesenliner 20 ist an dem nicht dargestellten Stumpf angelegt und in einen Prothesenschaft 10 eingeführt. Der Prothesenschaft 10 weist einen proximalen Rand 11 auf, der nicht in einer flachen Ebene liegt, sondern eine Raumkurve beschreibt. Der Prothesenschaft 10 weist medial und lateral hochgezogene Bereiche auf, die sich weiter in Proximalrichtung erstrecken als die Bereiche, die frontal und im Bereich der Kniekehle angeordnet sind. Frontal ist ein Ausschnitt zu erkennen, der es ermöglicht, dass die Kniescheibe beweglich ist. Im rückwärtigen Bereich der Kniekehle ist ein korrespondierender Ausschnitt oder eine korrespondierende Absenkung ausgebildet, um ein Einbeugen des Beines zu ermöglichen, ohne dass der Prothesenschaft mit seinem dorsalen Bereich zwischen dem rückwärtigen Oberschenkel und dem Wadenbereich eingeklemmt wird.

Der Prothesenliner 20 ist vollständig in einen Aufnahmeraum 15 des Prothesenschaftes 10 eingeführt, das heißt, dass das distale Ende 22 des Prothesenliners 20 im Bereich des distalen Endes 12 des Prothesenschaftes 10 befindlich ist, gegebenenfalls darauf aufliegt oder leicht beabstandet dazu, beispielsweise über ein Polster, daran angeordnet ist. Die Dichtlippe 26 liegt an der Innenwand des Prothesenschaftes 10 an und dichtet ein Volumen 30 zwischen der Innenwand des Prothesenschaftes 10 und der Außenwand 23 des Prothesenliners 20 distal zu der Dichtlippe 26 ab. Das Volumen 30 wird beispielsweise durch eine Pumpbewegung beim Gehen durch ein Auslassventil oder durch eine motorisch angetriebene Pumpe evakuiert, also auf ein Druckniveau gebracht, das unterhalb des Atmosphärendruckes liegt.

Der Figur 2 ist zu entnehmen, dass der Dichtlippenverlauf dem Verlauf des proximalen Randes 11 des Prothesenschaftes 10 entspricht oder diesem folgt und lediglich in Distalrichtung versetzt an der Außenseite des Grundkörpers 25 befindlich ist oder angeordnet ist. Idealerweise verläuft die Dichtlippe 26 in geringstmöglicher Entfernung zu dem proximalen Rand 11 des Prothesenschaftes 10. Insbesondere der Höhenverlauf oder die Höhenkontur, also der Verlauf der Dichtlippe 26 um den Umfang des Grundkörpers 25 in Proximal-Distal-Richtung, entspricht dem Höhenverlauf des proximalen Randes 11 des Prothesenschaftes. Geringe Abweichungen können möglich sein, insbesondere kann der Dichtlippenverlauf in einem Bereich festgelegt werden, der im Wesentlichen parallel zu dem Verlauf der Höhenkontur des proximalen Randes 11 des Prothesenschaftes 10 entspricht, wobei die proximale und distale Grenze des Bereiches zu dem Höhenkonturverlauf des proximalen Randes 11 korrespondierend ausgebildet ist.

Auch die Kontur in Umfangsrichtung, also die Kontur des Innenumfanges des Prothesenschaftes 10 im Bereich der Anlage der Dichtlippe 26 kann erfasst werden. Die Kontur des äußeren Umfanges der Dichtlippe 26 kann dann korrespondierend zu dem Verlauf der Umfangskontur im Bereich der Anlage des äußeren Dichtlippenrandes an der Innenseite des Prothesenschaftes 10 ausgebildet sein, versehen mit einem Zuschlag, sodass die Dichtlippe 26 an der Innenseite des Prothesenschaftes 10 mit einer leichten Vorspannung aufgrund der Rückstellkräfte bei einer Verformung nach dem Einführen des Prothesenliners 20 in dem Prothesenschaft 10 anliegen kann.

Alternativ zu einer Ausgestaltung des Prothesenschaftes 10 als Unterschenkelschaft mit Erhöhungen auf der medialen und lateralen Seite kann beispielsweise eine Ausgestaltung als Oberschenkelschaft eine nur einseitige Erhöhung lateral beinhalten, die ungefähr bis zur Drehachse des Hüftgelenkes reicht. Auf der medialen Seite des Oberschenkels ist dementsprechend ein in Distalrichtung versetzter Ausschnitt ausgebildet, sodass sich ein entsprechender Dichtlippenverlauf bei einem Oberschenkelliner einstellt.

Zur Herstellung eines solchen Liners 20 wird zunächst die Höhenkontur des Prothesenschaftes 10, der in der Regel individuell angefertigt wird, erfasst. Dazu wird auch die Höhe des Prothesenschaftes 10 erfasst, also der Abstand von dem proximalen Rand 11 bis zu dem distalen Ende 12 auf der Innenseite des Prothesenschaftes 10 über den Umfang des Stumpfes. Die Form und die Abmessungen können bevorzugt optisch erfasst werden, beispielsweise durch Bildaufnahmen und Bildauswertung, alternative Erfassungsdaten wie ein Abtasten oder ein Abfahren mit Messwertaufnehmern kann ebenfalls erfolgen. Wurde der Prothesenschaft 10 zur Herstellung bereits digital modelliert, so kann die Höhenkontur direkt aus dem digitalen Modell erfasst und als Referenz für den Dichtlippenverlauf verwendet werden.

Auf der Grundlage des erfassten Verlaufes der Höhenkontur des proximalen Randes 11 wird dann festgelegt, wo die Dichtlippe 26 an der Innenseite des Prothesenschaftes anliegen soll und damit wo die Dichtlippe an der Außenseite 23 des Grundkörpers 25 des Prothesenliners 20 anzuordnen ist. Die erfassten Daten werden dazu benutzt, um ein 3D-Datenmodell zu erstellen. Anhand des Datenmodells des Prothesenschaftes 10 wird der Liner 20 konstruiert, beispielsweise mit einem Standardgrundkörper 25 und einen individuellen Dichtlippenverlauf der Dichtlippe 26, der an dem Verlauf des proximalen Randes 11 des Prothesenschaftes 10 orientiert ist. Die Form des Prothesenliners 20 mit dem angepassten Dichtlippenverlauf wird ebenfalls als 3D-Datenmodell errechnet. Anhand des 3D-Datenmodells werden Fertigungsdaten erzeugt, anhand derer mit einem additiven Fertigungsverfahren der Prothesenliner 20 mit dem Dichtlippenverlauf korrespondierend zu dem Verlauf des proximalen Randes 11 des Prothesenliners gefertigt wird.

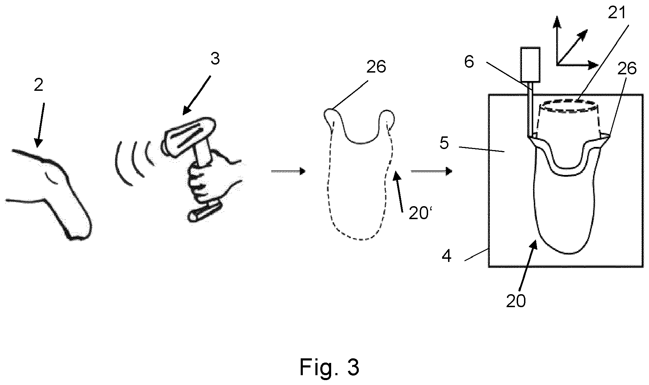

In der Figur 3 ist ein möglicher Ablauf eines Verfahrens zum Herstellen eines Prothesenliners 20 dargestellt. Von einem Stumpf 2, vorliegend einem Unterschenkelstumpf, wird über ein optisches Erfassungsgerät 3 die äußere Kontur des Stumpfes 2 aufgenommen, z.B. gescannt. Von dem Stumpf 2 wird ein 3D-Modell erstellt und in einem nicht dargestellten Rechner aufbereitet. Anhand des 3D-Modells wird ein Datensatz 20' errechnet, der im Wesentlichen die Form des späteren Prothesenliners 20 repräsentiert. Der Datensatz 20' legt neben dem Dichtlippenverlauf 26 auch die äußere Kontur des Prothesenliners 20 fest, insbesondere auch das distale Ende 22 sowie ggf. die Materialstärke des Prothesenliners 20. Über den Datensatz 20' ist es möglich, Verstärkungen, Materialschwächungen sowie die Verwendung unterschiedlicher Materialien zu definieren, die dann während des Fertigungsverfahrens verwendet oder eingearbeitet werden. Anhand des Datensatzes 20' kann der tatsächliche Prothesenliner 20 gefertigt werden. Der proximale Rand 21 beziehungsweise der Verlauf des proximalen Randes 21 des tatsächlichen Prothesenschaftes 20 ist in dem Ausführungsbeispiel noch nicht als im Datensatz 20' festgelegt eingezeichnet. Die übrige Kontur des Prothesenliners 20 ist durch die unterbrochene Linie angedeutet. Anhand des Datensatzes 20' oder der Grunddaten des Scanns kann ein Datensatz für den Prothesenschaft erstellt werden, der die Grundlage für dessen Fertigung bildet, beispielsweise in einem additiven Fertigungsverfahren. Der Dichtlippenverlauf 26 im Raum ist als Konturlinie definiert und kann als Referenz für den Verlauf der Kontur des proximalen Randes des Prothesenschaftes dienen. Der Dichtlippenverlauf der Dichtlippe 26 an der Außenseite eines noch zu fertigenden Prothesenliners 20 kann also zuerst bestimmt werden, anschließend wird der Prothesenschaft gestaltet. Es besteht umgekehrt die Möglichkeit, den Dichtlippenverlauf an einer bereits festgelegten Kontur des proximalen Randes eines virtuellen oder bereits bestehenden Prothesenschaftes anzupassen.

Anhand des Datensatzes 20' wird mit einem additiven Fertigungsverfahren der Prothesenliner 20 gefertigt. In dem dargestellten Ausführungsbeispiel erfolgt die Herstellung nach dem sogenannten rapid-liquid-printing-Verfahren, bei dem ein Stützmaterial 5 in einem Tank oder Vorratsbehälter 4 angeordnet ist. Über eine Düse 6, die dreidimensional im Raum verfahrbar ist, wird das Material des Prothesenliners 20 in das Stützmaterial 5 eingebracht und der Prothesenliner 20 additiv gefertigt. Die gestrichelte Linie deutet die proximale Endkontur des Prothesenliners 20 an, die in dem dargestellten Ausführungsbeispiel geradlinig ist. Die proximale Endkontur oder der proximale Rand 21 des Prothesenliners 20 kann auch korrespondierend zu dem Verlauf der Dichtlippe 26 oder korrespondierend zu dem proximalen Rand des Prothesenschaftes verlaufen.

## Patentansprüche

1. Verfahren zum Herstellen eines Prothesenliners (20) zum Einsatz in einem Prothesenschaft (10), der einen Aufnahmeraum (15) mit einem distalen Ende (12) und einem proximalen Rand (11) für einen Stumpf (2) und den Prothesenliner (20) aufweist, mit den Schritten:
a. Festlegen eines Dichtlippenverlaufes an der Außenseite (23) des Prothesenliners (20) korrespondierend zu dem Verlauf einer Höhenkontur des proximalen Randes (11) des Prothesenschaftes (10) oder anhand vorhandener, bekannter anatomischer Gegebenheiten des Stumpfes (2);
b. Anordnen einer Dichtlippe (26) an der Außenseite des Prothesenliners (20) entlang des festgelegten Dichtlippenverlaufes, **dadurch gekennzeichnet, dass** die Dichtlippe (26) einen Dichtlippenverlauf in Gestalt einer Raumkurve ausbildet.

2. Verfahren nach Anspruch 1, **dadurch gekennzeichnet, dass** vor dem Festlegen des Dichtlippenverlaufes die Höhenkontur des proximalen Randes (11) des Prothesenschaftes (10) erfasst wird.

3. Verfahren nach Anspruch 1 oder 2, **dadurch gekennzeichnet, dass** der Prothesenliner (20) additiv gefertigt wird oder die Dichtlippe (26) entlang des festgelegten Dichtlippenverlaufes festgelegt wird.

4. Verfahren nach einem der voranstehenden Ansprüche, **dadurch gekennzeichnet, dass** die Dichtlippe (26) in distaler Richtung zu dem proximalen Rand (11) des Prothesenschaftes (10) versetzt an dem Prothesenliner (20) angeordnet ist.

5. Verfahren nach einem der voranstehenden Ansprüche, **dadurch gekennzeichnet, dass** der Verlauf der Höhenkontur in proximal-distal-Richtung und/oder einer Umfangskontur über den Umfang des Prothesenschaftes (10) erfasst wird.

6. Verfahren nach einem der voranstehenden Ansprüche, **dadurch gekennzeichnet, dass** die Dichtlippe (26) über den Umfang des Prothesenliners (20) in proximal-distal-Richtung gleichbeabstandet zu dem proximalen Rand (11) des Prothesenschaftes (10) angeordnet wird.

7. Verfahren nach einem der voranstehenden Ansprüche, **dadurch gekennzeichnet, dass** die Dichtlippe (26) in einem Dichtlippenbereich angeordnet wird, der breiter als die Dichtlippe (26) ist und dessen proximale und distale Grenze zu der Höhenkontur des proximalen Randes (11) des Prothesenschaftes (10) korrespondiert.

8. Verfahren nach Anspruch 7, **dadurch gekennzeichnet, dass** der Dichtlippenbereich doppelt so breit wie die Dichtlippe (26) an ihrem Übergang zu einem Prothesenlinergrundkörper (25) ist.

9. Verfahren nach einem der voranstehenden Ansprüche, **dadurch gekennzeichnet, dass** die Höhenkontur oder die Höhen- und Umfangskontur des Prothesenschaftes (10) optisch erfasst wird, ein digitales 3D-Modell erstellt wird und der Dichtlippenverlauf oder der Dichtlippenverlauf und die Dichtlippenhöhe in Abhängigkeit von der erfassten Höhenkontur oder der erfassten Umfangs- und Höhenkontur festgelegt wird.

10. Verfahren nach Anspruch 9, **dadurch gekennzeichnet, dass** die Dichtlippenhöhe in Abhängigkeit von einen erfassten Abstand zwischen einem Innenumfang des Prothesenschaftes (10) und einem Außenumfang eines einzuführenden Stumpfes (2) festgelegt wird.

11. Verfahren nach Anspruch 3, **dadurch gekennzeichnet, dass** der Prothesenliner (20) in einem rapid-liquid-printing-Verfahren hergestellt wird.

12. System aus einem Prothesenschaft (10) und einem Prothesenliner (20), der Prothesenschaft (10) weist einen Aufnahmeraum für einen mit dem Prothesenliner (20) versorgten Stumpf (2) auf, der Prothesenschaft (10) weist ein distales Ende (12) und einen proximalen Rand (11) auf, der proximale Rand (11) des Prothesenschaftes (10) weist eine Höhenkontur und eine Umfangskontur auf, an der dem Prothesenschaft (10) zugewandten Außenseite (23) des Prothesenliners (20) ist zumindest eine Dichtlippe (26) ausgebildet oder festgelegt, der Dichtlippenverlauf der zumindest einen Dichtlippe (26) korrespondiert in dem vollständig eingeführten Zustand des Prothesenliners (20) mit dem Verlauf der Höhenkontur des Prothesenschaftes (10), **dadurch gekennzeichnet, dass** die Dichtlippe (26) einen Dichtlippenverlauf in Gestalt einer Raumkurve ausbildet.

13. System nach Anspruch 12, **dadurch gekennzeichnet, dass** der Prothesenschaft (10) distal zu der zumindest einen Dichtlippe (26) im vollständig eingeführten Zustand des Prothesenliners (20) geschlossenwandig ausgebildet ist.

14. System nach Anspruch 12 oder 13, **dadurch gekennzeichnet, dass** der Prothesenschaft (10) formstabil ausgebildet ist.

15. System nach einem der Ansprüche 12 bis 14, **dadurch gekennzeichnet, dass** die zumindest eine Dichtlippe (26) an einem Grundkörper (25) befestigt oder ausgebildet ist und eine über den Umfang des Prothesenliners (20) ungleichmäßige Breite aufweist.

16. System nach einem der Ansprüche 12 bis 15, **dadurch gekennzeichnet, dass** der Dichtlippenverlauf nicht in einer Ebene liegt.

17. Prothesenliner (20) für ein System nach einem der Ansprüche 12 bis 16, mit zumindest einer an einer Außenseite des Prothesenliners (20) ausgebildeten oder festgelegten Dichtlippe (26), die einen Dichtlippenverlauf in Gestalt einer Raumkurve ausbildet.

18. Prothesenliner (20) nach Anspruch 17, **dadurch gekennzeichnet, dass** der Dichtlippenverlauf der zumindest einen Dichtlippe (26) zu dem Verlauf einer Höhenkontur eines proximalen Randes (11) eines Prothesenschaftes (10) korrespondierend ausgebildet ist.

## Claims

1. A method for producing a prosthesis liner (20) for use in a prosthesis socket (10) which has a receiving space (15), with a distal end (12) and a proximal edge (11), for a stump (2) and the prosthesis liner (20), said method having the following steps:
a. determining a sealing lip profile on the outer face (23) of the prosthesis liner (20) corresponding to the profile of a height contour of the proximal edge (11) of the prosthesis socket (10) or based on existing, known anatomical characteristics of the stump (2);
b. arranging a sealing lip (26) on the outer face of the prosthesis liner (20) along the determined sealing lip profile, **characterized in that** sealing lip forms a sealing lip profile in the form of a three-dimensional curve.

2. The method as claimed in claim 1, **characterized in that** the height contour of the proximal edge (11) of the prosthesis socket (10) is detected before the sealing lip profile is determined.

3. The method as claimed in claim 1 or 2, **characterized in that** the prosthesis liner (20) is additively manufactured, or the sealing lip (26) is fixed along the determined sealing lip profile.

4. The method as claimed in one of the preceding claims, **characterized in that** the sealing lip (26) is arranged on the prosthesis liner (20) in a manner offset in the distal direction with respect to the proximal edge (11) of the prosthesis socket (10).

5. The method as claimed in one of the preceding claims, **characterized in that** the profile of the height contour is detected in the proximal-distal direction and/or the profile of a circumferential contour is detected over the circumference of the prosthesis socket (10).

6. The method as claimed in one of the preceding claims, **characterized in that** the sealing lip (26) over the circumference of the prosthesis liner (20) is arranged at the same distance in the proximal-distal direction from the proximal edge (11) of the prosthesis socket (10).

7. The method as claimed in one of the preceding claims, **characterized in that** the sealing lip (26) is arranged in a sealing lip region which is wider than the sealing lip (26) and of which the proximal and distal border corresponds to the height contour of the proximal edge (11) of the prosthesis socket (10) .

8. The method as claimed in claim 7, **characterized in that** the sealing lip region is twice as wide as the sealing lip (26) at its transition to a base body (25) of the prosthesis liner.

9. The method as claimed in one of the preceding claims, **characterized in that** the height contour or the height and circumferential contour of the prosthesis socket (10) is detected optically, a digital 3D model is created, and the sealing lip profile or the sealing lip profile and the sealing lip height is determined as a function of the detected height contour or of the detected circumferential and height contour.

10. The method as claimed in claim 9, **characterized in that** the sealing lip height is determined as a function of a detected distance between an inner circumference of the prosthesis socket (10) and an outer circumference of a stump (2) that is to be inserted.

11. The method as claimed in claim 3, **characterized in that** the prosthesis liner (20) is produced in a rapid liquid printing process.

12. A system composed of a prosthesis socket (10) and of a prosthesis liner (20), the prosthesis socket (10) has a receiving space for a stump (2) fitted with the prosthesis liner (20), the prosthesis socket (10) has a distal end (12) and a proximal edge (11), the proximal edge (11) of the prosthesis socket (10) has a height contour and a circumferential contour, at least one sealing lip (26) is formed or determined on the outer face (23) of the prosthesis liner (20) directed toward the prosthesis socket (10), the sealing lip profile of the at least one sealing lip (26) corresponds, in the fully inserted state of the prosthesis liner (20), to the profile of the height contour of the prosthesis socket (10), **characterized in that** sealing lip forms a sealing lip profile in the form of a three-dimensional curve.

13. The system as claimed in claim 12, **characterized in that**, when the prosthesis liner (20) is in the fully inserted state, the prosthesis socket (10) is designed with a closed wall distally with respect to the at least one sealing lip (26).

14. The system as claimed in claim 12 or 13, **characterized in that** the prosthesis socket (10) is dimensionally stable.

15. The system as claimed in one of the claims 12 through 14, **characterized in that** the at least one sealing lip (26) is fastened or formed on a base body (25) and has a non-uniform width over the circumference of the prosthesis liner (20).

16. The system as claimed in one of claims 12 through 15, **characterized in that** the sealing lip profile does not lie in one plane.

17. A prosthesis liner (20) for a system as claimed in one of claims 12 through 16, with at least one sealing lip (26) which is formed or fastened on an outer face of the prosthesis liner (20) and which forms a sealing lip profile in the shape of a three-dimensional curve.

18. The prosthesis liner (20) as claimed in claim 17, **characterized in that** the sealing lip profile of the at least one sealing lip (26) is designed corresponding to the profile of a height contour of a proximal edge (11) of a prosthesis socket (10).

## Revendications

1. Procédé de fabrication d'une doublure prothétique (20) destinée à être utilisée dans une emboîture de prothèse (10) qui présente un espace de réception (15), ayant une extrémité distale (12) et un bord proximal (11), pour un moignon (2) et qui présente la doublure prothétique (20), comprenant les étapes consistant à :
a. définir d'un tracé d'une lèvre d'étanchéité sur le côté extérieur (23) de la doublure prothétique (20), correspondant au tracé d'un contour en hauteur du bord proximal (11) de l'emboîture de prothèse (10), ou à l'aide de données anatomiques existantes et connues du moignon (2) ;
b. agencer une lèvre d'étanchéité (26) sur le côté extérieur de la doublure prothétique (20) le long du tracé défini de la lèvre d'étanchéité,
**caractérisé en ce que** la lèvre d'étanchéité (26) présente un tracé sous la forme d'une courbe spatiale.

2. Procédé selon la revendication 1,
**caractérisé en ce que** l'on saisit le contour en hauteur du bord proximal (11) de l'emboîture de prothèse (10) avant de définir le tracé de la lèvre d'étanchéité.

3. Procédé selon la revendication 1 ou 2,
**caractérisé en ce que** la doublure prothétique (20) est réalisée par fabrication additive, ou la lèvre d'étanchéité (26) est fixée le long du tracé défini de la lèvre d'étanchéité.

4. Procédé selon l'une des revendications précédentes,
**caractérisé en ce que** la lèvre d'étanchéité (26) est agencée sur la doublure prothétique (20) de manière décalée en direction distale par rapport au bord proximal (11) de l'emboîture de prothèse (10).

5. Procédé selon l'une des revendications précédentes,
**caractérisé en ce que** le tracé du contour en hauteur dans la direction proximale-distale et/ou d'un contour périphérique est saisi sur le pourtour de l'emboîture de prothèse (10).

6. Procédé selon l'une des revendications précédentes,
**caractérisé en ce que** la lèvre d'étanchéité (26) est agencée sur le pourtour de la doublure prothétique (20) à équidistance du bord proximal (11) de l'emboîture de prothèse (10) dans la direction proximale-distale.

7. Procédé selon l'une des revendications précédentes,
**caractérisé en ce que** la lèvre d'étanchéité (26) est agencée dans une zone de lèvre d'étanchéité qui est plus large que la lèvre d'étanchéité (26) et dont les limites proximale et distale correspondent au contour en hauteur du bord proximal (11) de l'emboîture de prothèse (10).

8. Procédé selon la revendication 7,
**caractérisé en ce que** la zone de la lèvre d'étanchéité est deux fois plus large que la lèvre d'étanchéité (26) à sa transition vers un corps de base (25) de la doublure prothétique.

9. Procédé selon l'une des revendications précédentes,
**caractérisé en ce que** le contour en hauteur ou le contour en hauteur et périphérique de l'emboîture de prothèse (10) est saisi par voie optique, un modèle numérique 3D est créé, et le tracé de la lèvre d'étanchéité ou le tracé de la lèvre d'étanchéité et la hauteur de la lèvre d'étanchéité sont définis en fonction du contour en hauteur saisi ou du contour périphérique et en hauteur saisi.

10. Procédé selon la revendication 9,
**caractérisé en ce que** la hauteur de la lèvre d'étanchéité est définie en fonction d'une distance saisie entre un pourtour intérieur de l'emboîture de prothèse (10) et un pourtour extérieur d'un moignon (2) à insérer.

11. Procédé selon la revendication 3,
**caractérisé en ce que** la doublure prothétique (20) est fabriquée par un procédé d'impression liquide rapide.

12. Système composé d'une emboîture de prothèse (10) et d'une doublure prothétique (20),
dans lequel l'emboîture de prothèse (10) présente un espace de réception pour un moignon (2) pourvu de la doublure prothétique (20),
l'emboîture de prothèse (10) présente une extrémité distale (12) et un bord proximal (11),
le bord proximal (11) de l'emboîture de prothèse (10) présente un contour en hauteur et un contour périphérique,
au moins une lèvre d'étanchéité (26) est formée ou fixée sur le côté extérieur (23) de la doublure prothétique (20) tourné vers l'emboîture de prothèse (10), dans l'état complètement inséré la doublure prothétique (20), le tracé de ladite au moins une lèvre d'étanchéité (26) correspond au tracé du contour en hauteur de l'emboîture de prothèse (10),
**caractérisé en ce que** la lèvre d'étanchéité (26) présente un tracé sous la forme d'une courbe spatiale.

13. Système selon la revendication 12,
**caractérisé en ce que** dans l'état complètement inséré de la doublure prothétique (20), l'emboîture de prothèse (10) est réalisée avec une paroi fermée du côté distal par rapport à ladite au moins une lèvre d'étanchéité (26).

14. Système selon la revendication 12 ou 13,
**caractérisé en ce que** l'emboîture de prothèse (10) est de forme stable.

15. Système selon l'une des revendications 12 à 14,
**caractérisé en ce que** ladite au moins une lèvre d'étanchéité (26) est fixée ou formée sur un corps de base (25) et présente une largeur irrégulière sur le pourtour de la doublure prothétique (20).

16. Système selon l'une des revendications 12 à 15,
**caractérisé en ce que** le tracé de la lèvre d'étanchéité ne se trouve pas dans un plan.

17. Doublure prothétique (20) pour un système selon l'une des revendications 12 à 16, comprenant au moins une lèvre d'étanchéité (26) formée ou fixée sur un côté extérieur de la doublure prothétique (20), qui forme un tracé de lèvre d'étanchéité sous la forme d'une courbe spatiale.

18. Doublure prothétique (20) selon la revendication 17,
**caractérisée en ce que** le tracé de ladite au moins une lèvre d'étanchéité (26) est réalisé de manière à correspondre au tracé d'un contour en hauteur d'un bord proximal (11) d'une emboîture de prothèse (10).
